Europäisches Patentamt

European Patent Office

Office européen des brevets

(19)

(11) Numéro de publication : **0 143 689**
**B1**

(12) **FASCICULE DE BREVET EUROPÉEN**

(45) Date de publication du fascicule du brevet :
04.05.88

(51) Int. Cl.⁴ : **A 61 M  1/10**, A 61 M 25/00,
F 16 L 37/26, F 16 L 37/28

(21) Numéro de dépôt : 84402164.2

(22) Date de dépôt : 26.10.84

(54) Système de raccord rapide entre un vaisseau sanguin et une prothèse cardiaque.

(30) Priorité : 09.11.83 FR 8317807

(43) Date de publication de la demande :
05.06.85 Bulletin 85/23

(45) Mention de la délivrance du brevet :
04.05.88 Bulletin 88/18

(84) Etats contractants désignés :
DE GB IT

(56) Documents cités :
CH-A-   418 069
DE-C-   635 593
FR-A- 1 362 459
GB-A- 2 123 532
US-A- 3 919 722
US-A- 4 187 846

(73) Titulaire : **AEROSPATIALE Société Nationale Industrielle**
37, Boulevard de Montmorency
F-75781 Paris Cédex 16 (FR)

(72) Inventeur : **Chareire, Jean-Louis**
66, rue Aristide Briand
F-92300 Levallois (FR)

(74) Mandataire : Bonnetat, Christian et al
Cabinet PROPI Conseils 23 rue de Léningrad
F-75008 Paris (FR)

Jouve, 18, rue St-Denis, 75001 Paris, France

## Description

La présente invention concerne un système de raccord rapide entre un vaisseau sanguin et une prothèse cardiaque, comportant deux embouts annulaires coopérants, dont l'un est fixé à ladite prothèse et l'autre à l'extrémité dudit vaisseau sanguin. Un tel système est par exemple décrit dans le document US-A-3 919 722.

On sait que l'opération de greffage d'un cœur artificiel s'effectue schématiquement de la manière suivante :

- on branche l'opéré sur une circulation sanguine extra-corporelle et on obture temporairement les vaisseaux arrivant ou partant du cœur ;
- on découpe le cœur naturel à la limite entre les deux oreillettes et les deux ventricules, ainsi qu'au départ de l'aorte et de l'artère pulmonaire ;
- on fixe par couture à l'extrémité de chacune des deux artères et des deux oreillettes un embout de raccord rapide, le cœur artificiel étant pourvu d'embouts correspondants ;
- on présente le cœur artificiel et on met en place les quatre embouts de raccord rapide des vaisseaux sur ceux du cœur artificiel ;
- et on purge l'air du cœur artificiel et des oreillettes et artères, jusqu'à la zone mise en circulation extracorporelle.

L'opération de purge de l'air est actuellement la plus difficile et représente plus de la moitié du temps de l'opération. De plus, il n'est jamais certain qu'elle soit effectuée de manière parfaite, ce qui fait courir un grand risque à l'opéré : en effet, une très petite quantité d'air parvenant au cerveau peut entraîner la mort ou des lésions irréversibles très graves.

L'invention a donc pour objet principal de rendre rapide et fiable l'opération de purge d'air et de prévoir un système de raccord rapide pour ce faire.

A cette fin, selon l'invention, le système de raccord rapide entre un vaisseau sanguin et une prothèse cardiaque, comportant deux embouts annulaires coopérants, dont l'un est fixé à ladite prothèse et l'autre à l'extrémité dudit vaisseau sanguin, est caractérisé en ce que :

- un soufflet tubulaire réalisé en un matériau imperméable au sang et perméable à l'air est prévu entre ledit vaisseau sanguin et l'embout de raccord rapide correspondant ;
- la partie extérieure de l'embout fixé à la prothèse forme une collerette susceptible d'être retenue prisonnière entre une paire de couronnes coaxiales et parallèles portées par l'embout fixé audit vaisseau sanguin, la couronne de ladite paire se trouvant vers l'extérieur étant ouverte pour permettre la solidarisation par coulissement desdits embouts ;
- une plaquette d'obturation amovible est montée sur ledit embout fixé à la prothèse cardiaque de façon à pouvoir coulisser dans un plan au moins sensiblement orthogonal à l'axe dudit embout, cette plaquette, d'une part, étant logée et guidée dans un évidement plat pratiqué dans la face de ladite collerette dirigée vers l'extérieur, ledit évidement débouchant à la périphérie de ladite collerette et, d'autre part, comportant un prolongement vers l'extérieur permettant de la saisir, lorsqu'elle se trouve dans sa position d'obturation ;

- ledit embout fixé audit vaisseau sanguin est pourvu d'un joint à débattement susceptible, lorsque les embouts sont solidarisés l'un de l'autre, d'assurer l'étanchéité entre ceux-ci, ainsi que de verrouiller lesdits embouts dans leur position de solidarisation, si ladite plaquette est séparée par coulissement dudit embout qui la porte, ledit joint à débattement comportant une partie tubulaire coaxiale auxdites couronnes de l'embout fixé audit vaisseau sanguin et une partie annulaire orthogonale à l'axe de ladite partie tubulaire, cette partie annulaire étant disposée du côté de la face intérieure de la couronne intérieure de ladite paire et étant ancrée à sa périphérie audit embout fixé au vaisseau, tandis que ladite partie tubulaire passe dans le trou central de ladite couronne intérieure pour faire saillie entre lesdites couronnes et, lorsque les embouts sont solidarisés l'un de l'autre, l'extrémité libre de ladite partie tubulaire, d'une part, s'appliquant sur ladite plaquette, si celle-ci est dans sa position d'obturation ou dans une position d'obturation partielle et, d'autre part, s'appliquant sur le fond dudit évidement plat servant de logement à la plaquette en s'accrochant derrière le bord de celui-ci, si ladite plaquette est séparée de l'embout qui la porte.

Avantageusement, ladite plaquette d'obturation est transparente.

Ainsi, comme on le verra plus en détail par la suite, la structure du système de raccord rapide selon l'invention :

- permet le remplissage du cœur artificiel juste avant l'opération par le sang du malade additionné d'anticoagulant ;
- permet la vérification parfaite du remplissage du cœur grâce aux plaquettes d'obturation transparentes laissant voir les bulles d'air éventuelles ;
- permet la fixation sur le cœur artificiel des quatre embouts liés aux vaisseaux sanguins ;
- facilite la purge des volumes compris entre lesdits embouts et les obturations temporaires des veines et artères ; et
- permet simultanément la mise en communication des volumes sanguins contenus dans le cœur artificiel et les embouts liés aux vaisseaux jusqu'aux obturations temporaires de ceux-ci et le verrouillage des embouts sur le cœur, sans reprise d'air.

De préférence, ladite plaquette d'obturation amovible se déplace parallèlement à une direction radiale dudit embout fixé à la prothèse cardiaque et lesdits moyens de raccord réciproque desdits embouts permettent de solidariser ceux-ci l'un de l'autre par un mouvement de coulissement de direction parallèle à la direction radiale de coulissement de ladite plaquette.

Avantageusement, le bord dudit évidement plat et le bord correspondant de ladite plaquette sont conformés en queue d'aronde s'élargissant vers l'intérieur de l'embout fixé à la prothèse cardiaque.

Les figures du dessin annexé feront bien comprendre comment l'invention peut être réalisée.

La figure 1 illustre schématiquement un cœur artificiel et deux vaisseaux sanguins qui doivent lui être reliés.

La figure 2 montre, en coupe axiale, un embout de raccord selon l'invention, devant être fixé sur un vaisseau sanguin à raccorder au cœur artificiel.

La figure 3 est une vue en bout, selon la flèche III de la figure 2, de l'embout montré par celle-ci.

La figure 4 montre, en coupe axiale, un embout de raccord selon l'invention, porté par le cœur artificiel.

La figure 5 est une vue en bout, selon la flèche V de la figure 4, de l'embout montrée par celle-ci.

La figure 6 illustre la solidarisation des embouts des figures 2 et 4, la plaquette d'obturation de l'embout porté par le cœur artificiel étant en place.

La figure 7 est une coupe suivant le plan VII-VII de la figure 6.

La figure 8 illustre la solidarisation des embouts des figures 2 et 4, la plaquette d'obturation de l'embout porté par le cœur artificiel étant éliminée.

La figure 9 est une coupe suivant le plan IX-IX de la figure 8.

La figure 10 illustre un procédé de raccord de la prothèse selon l'invention.

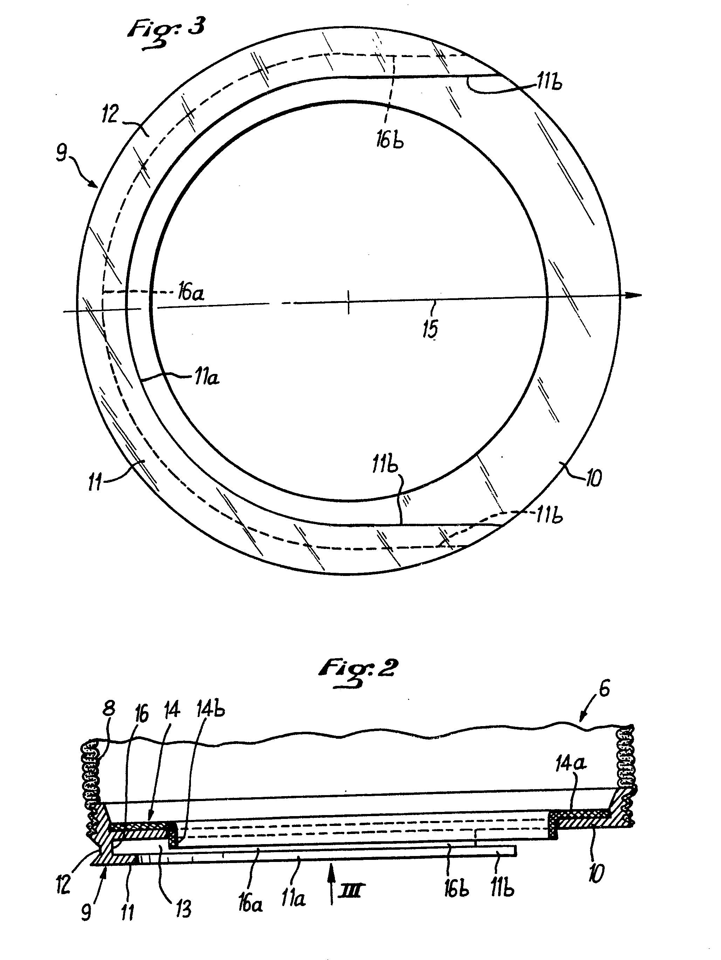

Sur ces figures, des références identiques désignent des éléments semblables. Les figures 2 à 9 sont des vues agrandies d'un mode de réalisation de l'invention, à une échelle environ égale à quatre. De façon connue, les embouts liés à la prothèse cardiaque comportent une soupape ne faisant pas partie de la présente invention et dont le sens dépend si ledit embout est destiné au raccord d'une veine ou d'une artère. Au sens près de ladite soupape, le système selon l'invention montré par les figures 2 à 8 est utilisable aussi bien pour le raccord à une veine que pour le raccord à une artère.

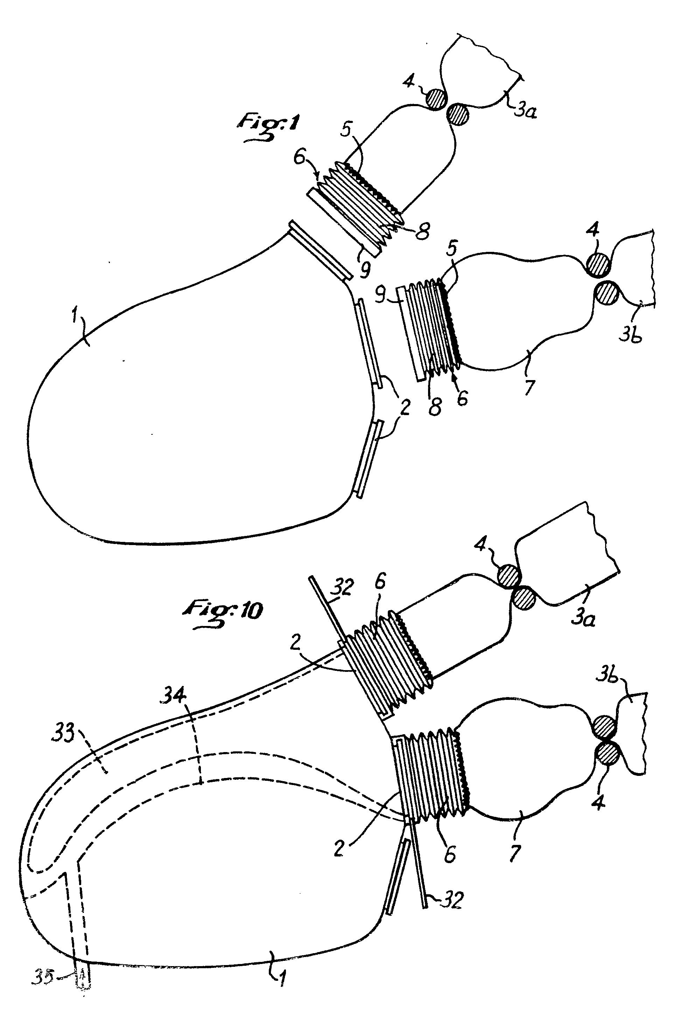

Sur la figure 1, on a représenté une prothèse cardiaque ou cœur artificiel 1 pourvu de quatre embouts 2 (dont trois seulement sont visibles) et devant être reliés à deux artères 3a, à savoir l'aorte et l'artère pulmonaire, (dont une seule est représentée) et à deux veines 3b, à savoir la veine cave et la veine pulmonaire (dont une seule est représentée).

Pour le greffage de la prothèse 1, le chirurgien branche l'opéré sur une circulation sanguine extra-corporelle (non représentée) et obture temporairement les artères 3a et les veines 3b au moyen de dispositifs à pincements 4. Ensuite, il découpe le cœur naturel (non représenté), par exemple à la limite entre les deux oreillettes et les deux ventricules, ainsi qu'au départ de l'aorte et de l'artère pulmonaire. Il fixe, par des coutures 5, des embouts 6 aux extrémités coupées des artères 3a et des oreillettes 7, ces embouts 6 étant chacun susceptibles de coopérer avec l'un des embouts 2 du cœur 1 pour former un système de raccord rapide. Chacun des embouts 6 comporte un soufflet tubulaire 8, en une matière hémocompatible telle que le DACRON (marque déposée), servant à le relier au vaisseau 2 ou 3 correspondant et une collerette 9, par exemple en titane, servant à le fixer sur un embout 2.

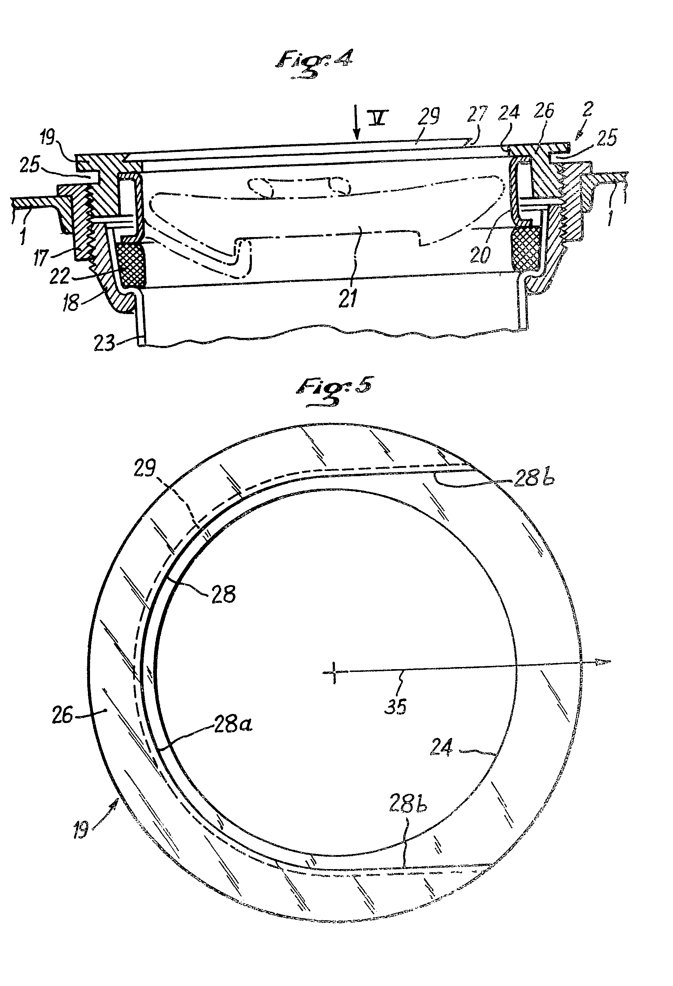

Comme le montrent les figures 2 et 3, la collerette 9 est constituée de deux couronnes plates, parallèles et coaxiales 10 et 11, solidaires l'une de l'autre à leur périphérie par une jante 12 et espacées pour ménager entre elles un espace plat 13, en forme d'un disque ayant un diamètre supérieur aux diamètres intérieurs desdites couronnes. Le soufflet 8 est solidarisé de la collerette 9 par l'intermédiaire de la jante 12.

La couronne intérieure 10, disposée du côté du soufflet 8, présente un diamètre intérieur plus petit que celui de la couronne extérieure 11. Un joint élastique 14, par exemple en DELRAIN (marque déposée), coaxial aux couronnes 10 et 11, comporte une partie annulaire 14a, qui s'applique sur la face de la couronne 10 dirigée vers le soufflet 8 et qui est ancrée par sa périphérie extérieure dans la face interne de la jante 12, ainsi qu'une partie tubulaire 14b, orthogonale à ladite partie annulaire 14a, et coaxiale aux couronnes 10 et 11, faisant saillie à l'intérieur dudit espace 13 en direction de la couronne extérieure 11. La face extérieure de la partie tubulaire 14b épouse sensiblement le contour de l'évidement intérieur de la couronne intérieure 10.

Par ailleurs, la couronne extérieure 11 et la jante 12 sont éliminées par usinage sur environ la moitié de leur périphérie, de façon que l'espace 13 soit ouvert parallèlement à un rayon 15 de la collerette 9. Cet usinage est effectué de façon que la périphérie intérieure de la couronne extérieure 11 soit constituée d'une partie semi-circulaire 11a, à laquelle sont raccordées deux parties rectilignes 11b, parallèles au rayon 15. De même, le fond périphérique 16 de l'espace plat 13 comporte une partie semi-circulaire 16a, à laquelle sont raccordées deux parties rectilignes 16b parallèles au rayon 15.

Comme on peut le voir sur les figures 4 et 5, un embout 2 comporte une bague annulaire 17, filetée intérieurement et solidaire de la prothèse 1. Dans la bague annulaire 17 sont vissés, par leur périphérie extérieure, deux anneaux 18 et 19, maintenant entre eux le corps cylindrique 20 de la soupape 21, mentionnée ci-dessus, ainsi qu'une bague élastique 22, servant à presser une poche à sang 23 constituant une partie active de la prothèse 1.

La bague 17, les anneaux 18 et 19 et le corps 20 peuvent être en titane, tandis que la bague élastique 22 et la poche à sang 23 peuvent être respectivement réalisées en TEFLON (marque déposée) et en polyuréthane.

La partie extérieure de l'anneau 19 est percée d'un trou intérieur 24 dont le diamètre est au plus égal au diamètre intérieur de la partie tubulaire 14b du joint 14. Une gorge 25 est usinée dans la paroi latérale dudit anneau 19 de façon à délimiter vers l'extérieur une couronne plate 26. On fait en sorte que :

- le diamètre extérieur et l'épaisseur de la couronne plate 26 soient légèrement inférieurs au diamètre et à la hauteur de l'espace 13 en forme de disque de l'embout 6 ;

- le diamètre de fond de la gorge 25 soit légèrement inférieur au diamètre intérieur de la couronne 11 de l'embout 6 ;

- la largeur de la gorge 25 soit légèrement supérieure à l'épaisseur de la couronne 11 de l'embout 6.

Ainsi, la partie supérieure de l'anneau 19 forme une sorte de collerette qui peut être emboîtée latéralement dans la collerette 9 de l'embout 6 par coulissement latéral à travers l'ouverture de la couronne 11. En position emboîtée des deux collerettes 25, 26 et 9, c'est-à-dire en position de raccord des embouts 2 et 6, comme montré sur les figures 6 à 9, la couronne plate 26 est logée dans la partie périphérique de l'espace 13 et maintenue par les couronnes 10 et 11.

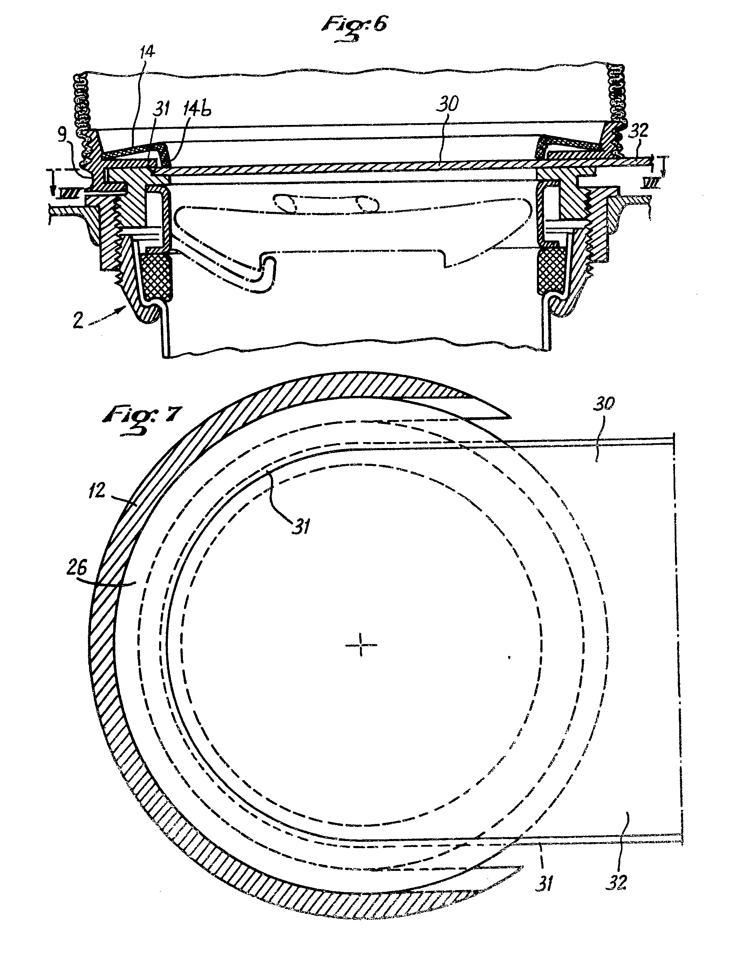

Par ailleurs, dans la face supérieure de la collerette plate 26 est usiné un évidement plat 27 délimité par un bord 28 formé par un demi-cercle 28a de rayon supérieur à celui du trou intérieur 24 de l'anneau 19 et au moins égal au rayon extérieur de la partie tubulaire 14b du joint 14 et par deux portions de droites 28b faisant déboucher ledit évidement jusqu'à la périphérie de ladite collerette 26. Le bord 28 est conformé en queue d'aronde 29 allant en s'élargissant de l'extérieur vers l'intérieur de l'embout 2. Ainsi, le bord 28 peut former coulisse pour une plaquette transparente 30, par exemple en PLEXIGLASS ou en LEXAN (marques déposées), non représentée sur les figures 4 et 5, mais visible sur les figures 6 et 7. Le mouvement de coulissement de la plaquette 30 s'effectue parallèlement au rayon 35 de l'anneau 19 parallèle aux portions de droites 28b.

Le bord avant de la plaquette 30 est délimité par une queue d'aronde 31 coopérant avec la queue d'aronde 29 du bord 28. La plaquette 30 se prolonge à l'extérieur de l'embout 2 par un prolongement 32.

En vue de la greffe de la prothèse cardiaque 1 selon l'invention, on commence par emplir ladite prothèse de sang et on obture les différents embouts 2 au moyen desdites plaquettes 30. On remarquera que grâce à la transparence de celles-ci, il est facile de s'assurer que ce remplissage est complet et qu'aucune bulle d'air n'est présente à l'intérieur de la prothèse.

Ensuite, les embouts 6 ayant été fixés sur les artères et veines 3a et 3b, on met en place la prothèse remplie de sang et on raccorde les différents embouts 6 aux différents embouts 2 correspondants, de la manière qui a été indiquée ci-dessus, par coulissement parallèle au plan et à la direction 35 du prolongement 32 de chaque plaquette 30. On se trouve alors dans la situation représentée sur les figures 6 et 7, dans laquelle la partie tubulaire 14b de chaque joint 14 est repoussée, par la plaquette 30 correspondante, vers l'intérieur de l'embout 6.

A partir de ce moment, le chirurgien doit effectuer l'opération de purge des cavités délimitées entre les paires d'embouts 2, 6 et les dispositifs 4 de pincement veineux et artériels (voir également la figure 10).

Ce chirurgien a le choix entre deux procédés :

a) soit le procédé classique à la seringue effectué en desserrant progressivement les dispositifs 4 ;

b) soit un procédé qui met en œuvre le principe de pompage du cœur artificiel 1. Pour expliquer ce principe, on a représenté schématiquement sur la figure 10 la poche à sang 33 d'un des ventricules et une membrane interne rigide 34 séparant les deux ventricules. La compression de la poche à sang 33 s'effectue sur introduction de gaz comprimé en 35.

Donc, si l'on place ce ventricule sous légère pression de gaz et que l'on tire très légèrement l'une des plaquettes vers l'extérieur, il s'établit une communication du cœur avec l'espace à purger de sorte que le sang contenu dans le cœur 1 jaillit dans cet espace, sans que l'on ait à desserrer les dispositifs de pincement 4 et sans risque de faire rentrer de l'air dans le cœur.

Le niveau du sang monte lentement dans l'espace à purger et la pression de l'air qui y est occlue augmente.

Or, les soufflets souples 8 en DACRON sont imperméables au sang mais perméables à l'air.

Il suffit donc de maintenir constamment les soufflets 8 dans la position la plus élevée pour que la purge soit complète.

L'avantage de ce procédé sur le premier est que l'on dispose ainsi d'une pression de sang beaucoup plus élevée et que la purge est plus complète et plus rapide.

Après la purge, effectuée pour l'un ou l'autre de ces procédés, le chirurgien va tirer successivement et d'un geste rapide chacune des quatre plaquettes 30 équipant le cœur, en les saisissant par leur prolongement 32.

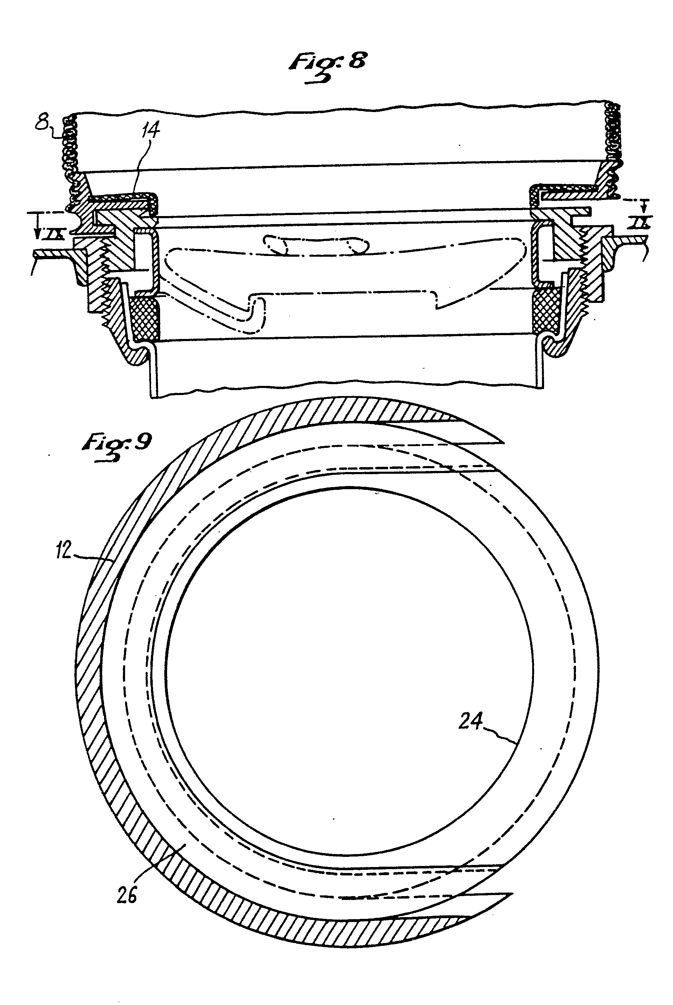

On voit que, de cette façon (figures 8 et 9) le joint-verrouilleur 14 est brutalement libéré.

Il vient prendre appui par sa partie tubulaire 14b sur le fond de l'évidement plat 27 et exerce sur celui-ci une force constante.

L'étanchéité est donc immédiatement et durablement assurée sans aucune reprise d'air.

De plus, ce joint 14 empêche le retour en arrière de l'embout 6 par rapport à l'embout 2 en rendant impossible le mouvement latéral par appui sur le sommet de la queue d'aronde 29. Le verrouillage est donc effectué.

Cependant, si un accident survenait à ce moment et qu'il faille à nouveau désolidariser le cœur 1 des quatre embouts 6, il suffirait de couper les coutures de liaison 5 de ces embouts avec les artères ou oreillettes et ensuite de faire tourner ceux-ci de 180° pour annuler l'effet de verrouillage du joint 14 et les désolidariser des embouts 2.

## Revendications

1. Système de raccord rapide entre un vaisseau sanguin (3a, 3b) et une prothèse cardiaque (1), comportant deux embouts annulaires coopérants (2, 6), dont l'un (2) est fixé à ladite prothèse et l'autre (6) à l'extrémité dudit vaisseau sanguin, caractérisé en ce que :
- un soufflet tubulaire (8) réalisé en un matériau imperméable au sang et perméable à l'air est prévu entre ledit vaisseau sanguin et l'embout de raccord rapide (6) correspondant ;
- la partie extérieure de l'embout (2) fixé à la prothèse (1) forme une collerette (26) susceptible d'être retenue prisonnière entre une paire de couronnes (10, 11) coaxiales et parallèles portées par l'embout (6) fixé audit vaisseau sanguin, la couronne (11) de ladite paire se trouvant vers l'extérieur étant ouverte pour permettre la solidarisation par coulissement desdits embouts ;
- une plaquette d'obturation amovible (30) est montée sur ledit embout (2) fixé à la prothèse cardiaque de façon à pouvoir coulisser dans un plan au moins sensiblement orthogonal à l'axe dudit embout, cette plaquette, d'une part, étant logée et guidée dans un évidement plat (27) pratiqué dans la face de ladite collerette (26) dirigée vers l'extérieur, ledit évidement (27) débouchant à la périphérie de ladite collerette et, d'autre part, comportant un prolongement (32) vers l'extérieur permettant de la saisir, lorsqu'elle se trouve dans sa position d'obturation,
- ledit embout (6) fixé audit vaisseau sanguin est pourvu d'un joint à débattement (14) susceptible, lorsque les embouts sont solidarisés l'un de l'autre, d'assurer l'étanchéité entre ceux-ci, ainsi que de verrouiller lesdits embouts dans leur position de solidarisation, si ladite plaquette est séparée par coulissement dudit embout qui la porte, ledit joint à débattement (14) comportant une partie tubulaire (14b) coaxiale auxdites couronnes (10, 11) de l'embout (6) fixé audit vaisseau sanguin et une partie annulaire (14a) orthogonale à l'axe de ladite partie tubulaire, cette partie annulaire (14a) étant disposée du côté de la face intérieure de la couronne intérieure (10) de ladite paire et étant ancrée à sa périphérie audit embout fixé au vaisseau, tandis que ladite partie tubulaire (14b) passe dans le trou central de ladite couronne intérieure (10) pour faire saillie entre lesdites couronnes (10 et 11) et, lorsque les embouts sont solidarisés l'un de l'autre, l'extrémité libre de ladite partie tubulaire (14b), d'une part, s'appliquant sur ladite plaquette (30), si celle-ci est dans sa position d'obturation ou dans une position d'obturation partielle et, d'autre part, s'appliquant sur le fond dudit évidement plat (27) servant de logement à la plaquette (30) en s'accrochant derrière le bord (28) de celui-ci, si ladite plaquette (30) est séparée de l'embout (2) qui la porte.

2. Système de raccord rigide selon la revendication 1, caractérisé en ce que ladite plaquette (30) est transparente.

3. Système de raccord rapide selon la revendication 1, caractérisé en ce que ladite plaquette d'obturation amovible (30) se déplace parallèlement à une direction radiale (35) dudit embout (2) fixé à la prothèse cardiaque et en ce que lesdits moyens de raccord réciproque desdits embouts permettent de solidariser ceux-ci l'un de l'autre par un mouvement de coulissement de direction parallèle à la direction radiale (35) de coulissement de ladite plaquette.

4. Système de raccord rapide suivant la revendication 1, caractérisé en ce que le bord (28) dudit évidement plat (27) et le bord correspondant de ladite plaquette sont conformés en queue d'aronde (29, 31) s'élargissant vers l'intérieur de l'embout fixé à la prothèse cardiaque.

## Claims

1. Quick connect system for connecting a blood vessel (3a, 3b) and a cardiac prosthesis (1), comprising two cooperating annular connectors (2, 6) of which one (2) is fixed to said prosthesis and the other (6) to the end of said blood vessel, characterized in that :
- a tubular bellows (8) made in a material which is impervious to blood and pervious to air is provided between said blood vessel and the corresponding connector (6) ;
- the outer part of the connector (2) fixed to the prosthesis (1) forms a flange (26) adapted to be held prisoner between a pair of coaxial, parallel rings (10, 11) borne by the connector (6) fixed to said blood vessel, the ring (11) of said pair located towards the outside being open in order to allow said connectors to be joined by sliding ;
- a removable obturating plate (30) is mounted on said connector (2) fixed to the cardiac prosthesis so as to be able to slide in a plane at least substantially orthogonal to the axis of said connector, this plate, on the one hand, being housed and guided in a flat recess (27) made in the face of said flange (26) directed outwardly, said recess (27) opening out on the periphery of said flange, and, on the other hand, comprising an outward extension (32) enabling it to be gripped, when it is in its position of obturation ;
- said connector (6) fixed to said blood vessel is provided with a seal (14) which, when the connectors are joined one to the other, ensures tightness therebetween, and which locks said connectors in their position of connection, if said plate is separated by sliding from said connector which bears it, the seal (14) comprising a tubular part (14b) coaxial to said rings (10, 11) of the connector (6) fixed to said blood vessel and an annular part (14a) at right angles to the axis of said tubular part, this annular part (14a) being disposed towards the inner face of the inner ring (10) of said pair and being anchored on its periphery on said connector fixed to the vessel, whilst said tubular part (14b) passes in the central hole of said inner ring (10) to project between said rings (10 and 11) and, when the connectors are joined one to the other, the free end of said tubular part (14b), on the one hand, being applied on said plate (30) if the latter is in its position of obturation or in a position of partial obturation and, on the other hand, being applied on the bottom of said flat recess (27) serving as housing for the plate (30) by hooking behind the edge (28) thereof, if said plate (30) is separated from the connector (2) which bears it.

2. Quick connect system according to claim 1, characterized in that said plate (30) is transparent.

3. Quick connect system according to claim 1, characterized in that the removable obturating plate (30) moves parallel to a radial direction (35) of said connector (2) fixed to the cardiac prosthesis and in that said means for reciprocal connection of said connectors enable the latter to be joined one to the other by a movement of slide in a direction parallel to the radial direction (35) of slide of said plate.

4. Quick connect system according to claim 1, characterized in that the edge (28) of said flat recess (27) and the corresponding edge of said plate are shaped as a dove-tail (29, 31) widening inwardly of the connector fixed to the cardiac prosthesis.


**Patentansprüche**

1. System zur schnellen Verbindung eines Blutgefäßes (3a, 3b) mit einer Herzprothese (1) mit zwei zusammenwirkenden ringförmigen Ansatzstücken (2, 6), von denen das eine (2) an der Prothese (1) und das andere am Ende des Blutgefäßes (3a, 3b) befestigt ist, dadurch gekennzeichnet, daß :

- ein rohrförmiger Balg (8) aus einem Material, das für Blut undurchlässig und für Luft durchlässig ist, zwischen dem Blutgefäß (3a, 3b) und dem entsprechenden Ansatzstück (6) zur schnellen Verbindung vorgesehen ist ;

- der äußere Teil des an der Prothese (1) befestigten Ansatzstücks (2) einen Bund (26) bildet, der zwischen einem Paar von koaxialen und parallelen, von am Blutgefäß (3a, 3b) befestigten Ansatzstück (6) getragenen Kronen (10, 11) festgehalten werden kann, wobei die Krone (11) dieses Paars, die nach außen gerichtet ist, derart geöffnet ist, daß sie eine durch Einschieben realisierte feste Verbindung der Ansatzstücke erlaubt ;

- eine bewegbare Absperrplatte (30) an dem an der Herzprothese (1) befestigten Ansatzstück (2) derart angebracht ist, daß sie in einer mindestens im wesentlichen senkrechten Ebene zur Achse des Ansatzstücks verschiebbar ist, wobei diese Platte (30) einerseits in einer ebenen, in der nach außen gerichteten Fläche des Bunds (26) untergebrachten Aussparung (27) sitzt und geführt ist, wobei sich die Aussparung (27) zur Peripherie des Bunds (26) öffnet, und wobei sie andererseits eine Verlängerung (32) nach außen aufweist, an der sie gefaßt werden kann, wenn sie sich in der Absperrstellung befindet ;

- das am Blutgefäß (3a, 3b) befestigte Ansatzstück (6) mit einer elastischen Verbindung (14) versehen ist, die, wenn die Ansatzstücke fest miteinander verbunden sind, eine Abdichtung ebenso wie eine Verriegelung zwischen diesen erlaubt, wenn die Platte (30) durch Verschieben vom sie tragenden Ansatzstück getrennt wird, wobei die elastische Verbindung (14) einen rohrförmigen, mit den Kronen (10, 11) des mit dem am Blutgefäß (3a, 3b) befestigten Ansatzstücks (6) koaxialen Teil (14b) und einen zur Achse des rohrförmigen Teils senkrechten, ringförmigen Teil (14a) umfaßt, wobei der ringförmige Teil (14a) auf der Seite der Innenfläche der inneren Krone (10) des Paares von Kronen angeordnet und mit seinem Umfang an dem am Gefäß befestigten Ansatzstück verankert ist, während der rohrförmige Teil (14b) durch das zentrale Loch der inneren Krone (10) verläuft und zwischen den Kronen (10 und 11) vorsteht, und wobei, wenn die Ansatzstücke fest miteinander verbunden sind, das freie Ende des rohrförmigen Teils (14b) einerseits auf der Platte (30) anliegt, falls sich diese In ihrer Absperrstellung oder in einer teilweisen Absperrstellung befindet, und andererseits auf dem Boden der ebenen Aussparung (27) anliegt, die als Sitz für die Platte (30) dient, indem sie sich hinter deren Rand (28) einhakt, wenn die Platte (30) von dem sie tragenden Ansatzstück (2) getrennt ist.

2. System zur schnellen Verbindung nach Anspruch 1, dadurch gekennzeichnet, daß die Platte (30) transparent ist.

3. System zur schnellen Verbindung nach Anspruch 1, dadurch gekennzeichnet, daß die bewegbare Absperrplatte (30) sich parallel in einer radialen Richtung (35) zum an der Herzprothese befestigten Ansatzstück (2) erstreckt, und daß die Mittel zum gegenseitigen Verbinden der Ansatzstücke es erlauben, diese fest miteinander über eine Verschiebebewegung in einer Richtung parallel zur radialen Richtung (35) der Verschiebung der Platte zu verbinden.

4. System zur schnellen Verbindung nach Anspruch 1, dadurch gekennzeichnet, daß der Rand (28) der ebenen Aussparung (27) und der entsprechende Rand der Platte (30) als Schwalbenschwanzführung (29, 31) ausgebildet sind, die sich zum Innern des an der Herzprothese befestigten Ansatzstücks erweitert.

*Fig. 1*

*Fig. 10*

*Fig. 3*

*Fig. 2*

2

*Fig. 4*

*Fig. 5*

Fig. 6

Fig. 7

*Fig. 8*

*Fig. 9*